# EUROPEAN PATENT APPLICATION

(11) **EP 4 321 872 A1**
(43) Date of publication of application: **14.02.2024**
(21) Application number: 23794226.3
(22) Date of filing: 14.06.2023
(51) Int. Cl.: G01N 33/68, G01N 15/14

(54) **NUCLEIC ACID APTAMER-BASED EXTRACELLULAR VESICLE FLUORESCENCE POLARIZATION DETECTION METHOD AND APPLICATION THEREOF**

(30) Priority: 28.06.2022 CN 202210738321
(71) Applicant: Shanghai Onetar Biomedicine Co., Ltd., Shanghai 200120 (CN)
(72) Inventor: DUAN, Wei, Shanghai 200120 (CN); XU, Changcheng, Shanghai 200120 (CN); FAN, Qiangyue, Shanghai 200120 (CN); XIANG, Songxi, Shanghai 200120 (CN)
(74) Representative: Isarpatent
(86) International application number: PCT/CN2023/100289
(87) International publication number: WO 2024/001798

(57) **Abstract**

The present invention relates to the technical field of C12N15/115, and particularly relates to an aptamer-based fluorescence polarization detection method for extracellular vesicles (EVs) and its application. The method comprises the following steps: S1. immobilizing EVs by interacting with antibodies against surface-biomarker proteins of EVs or surface cancer markers of EVs; rapidly washing them to remove free EVs, proteins, membrane fragments, and lipids; S2. respectively adding aptamers that are matched with EV markers or cancer cell markers therein and cultivating them the aptamers are fluorescently labeled; S3. performing fluorescence polarization detection on the products from Step S2 to achieve qualitative and quantitative analysis of EVs secreted by cancer cells. This invention can specifically detect extracellular vesicles secreted by cancer cells in blood, and the detection process is not interfered with by free tumor marker proteins, tumor cell membrane fragments, or tumor cell extracellular vesicle membrane fragments in the blood. The detection results are accurate and effective.

## Description

### TECHNICAL FIELD

The present invention relates to the technical field of C12N15/115 (aptamers that specifically and highly affine bind to target molecules), especially relaes to an aptamer-based fluorescence polarization detection method for extracellular vesicles and its application.

### DESCRIPTION OF THE RELATED ART

Lab-on-a-chip devices, Nanoparticle Tracking Analysis (NTA), high-resolution single Extracellular Vesicle (EV) analysis techniques, flow cytometry, and Enzyme-Linked Immunosorbent Assay (ELISA) are the main methods currently used to detect and quantify extracellular vesicles in a high-throughput manner. However, chip-based laboratory technologies such as microfluidics and microelectromechanical chips require advanced micromachining techniques and precision instruments to read signals and analyze data, thereby limiting the widespread application of these technologies in laboratory and clinical EV research. Highsensitivity flow cytometers are not only used to detect and quantify EVs but can also characterize individual EVs with high-throughput and multiplex analysis potential. Before this technology is widely applied to EV research and clinical practice, time costs, population effects, and moderate reproducibility will inevitably drive its extensive development. Although ELISA is a common biochemical detection method, the detection limit of ELISA for EVs is relatively high only within the range of 10⁹-10¹⁰ EVs/mL. Therefore, it is an urgent problem to be solved in laboratory and clinical fields to explore a simple, specific, and sensitive method for detecting and quantifying EVs.

In the decades following Weber's research, fluorescence polarization has been used in clinical environments and high-throughput analyses (i.e., drug discovery). The rapid development of this technology can be attributed to its good reproducibility, high degree of autonomy, adaptability to small volume samples (about 10µL), and its ability to not separate free and bound ligands in homogeneous analysis. Heterogeneous techniques that separate unbound and bound species are not only more laborious but also have issues with inaccurate quantification of actual biomolecular interactions. Meanwhile, other fluorescence-based homogeneous assays, such as Fluorescence Resonance Energy Transfer (FRET), Time-Resolved Fluorescence (TRF), or Time-Resolved FRET (TR-FRET), require multiple labels rather than a single label used in fluorescence polarization. Furthermore, as a ratio measurement method, fluorescence polarization can eliminate artifacts triggered by internal filtering effects, reduce the influence of the analytical environment, and be not affected by the absorbance or color quenching of other compounds.

In the development process of Fluorescence Polarization (FP) detection methods, it is a crucial factor to select probe substances with a molecular weight less than 15kDa. Based on this, lipophilic dyes such as PKH26 and PKH67, and carbocyanine dyes (Dil, DiO) serve as ligands; although their molecular weight is small (~1kDa), they per se have inherent limitations, such as non-specific binding with non-EV particles, low labeling efficiency, and additional effects when dye aggregates. While antibodies specifically bind to EV markers, their large size (about 150kDa) may not be suitable for FP. In contrast, aptamers (chemical antibodies) are much smaller in size (<15kDa) and are more stable in chemical synthesis, making them ideal probe substances for EV detection based on FP.

There are only two reports in the prior art on using FP to detect EVs. Kalimuthu et al. used 5-dodecylaminofluorescein (C12-FAM, a lipophilic dye) as the probe substance to detect EVs sourced from the HT29 cell line and TCMK1 cell line. However, dye molecule aggregation and the non-specific interactions between the probe substance (C12-FAM) and non-EV targets (such as membrane fragments or lipoproteins in EV solutions) interfere with the observation of FP signals. In another report (Z. Zhang et al., "Aptamer-based fluorescence polarization assay for separation-free exosome quantification", Nanoscale, 2019, 11, 10106-10113), a CD63 aptamer was used to directly detect EVs in the A549 cell line or human plasma without isolation of EVs in advance. Although the present applicant confirmed that the sensitivity of this experimental method to CD63-positive EVs is much higher than other soluble CD63 proteins, regardless of its use in cancer diagnosis, in the extraction of EVs or human plasma, the probe used will interact with soluble proteins and/or protein aggregates in EVs, resulting in positive detection results where the detected target is soluble proteins or membrane fragments rather than extracellular vesicles secreted from cancer cells, as the LOD of the aptamer's FP detection method can reach 86 aM or 28 aM.

The present application provides an aptamer-based fluorescence polarization detection method for extracellular vesicles (that is, Fluorescence Polarization using Aptamers for the Detection of Extracellular nanovesicles, FluPADE). Thanks to the high specificity of antibodies and aptamers, and the small size of aptamers, this method can efficiently and accurately detect oncogenic EVs in cell culture media or human plasma.

### SUMMARY

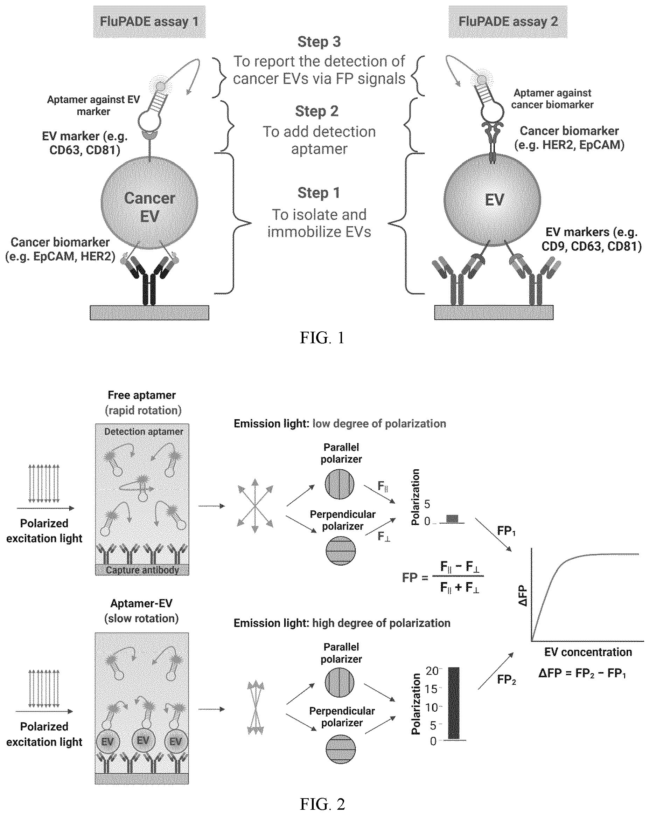

The first aspect of the invention provides a fluorescence polarization detection method for extracellular vesicles (EVs) based on (nucleic acid) aptamers, as shown in FIG. 1, which includes the following steps:
S1. immobilizing the EVs by interacting antibodies with biomarker proteins on the EV surface or cancer markers on the EV surface, then, rapidly washing them to remove free EVs, proteins, membrane fragments, and lipids;
S2. adding aptamers matched with the EV markers or cancer cell markers respectively for incubation, wherein the aptamers are subjected to fluorescent labeling;
S3. detecting the product obtained in step S2 by fluorescence polarization, thus realizing qualitative and quantitative analysis of the EVs secreted by cancer cells without washing during an operation process.

The biochemical processes and biophysical measurement principles in this detection method are shown in FIG. 2.

The EVs secreted by cancer cells are in fact inducers that allow the fluorescence polarization signal to manifest when using various biological markers to capture and detect the EVs. In the FluPADE detection of the present invention, three different aptamers are used to demonstrate that the detection method can be optimized through targeting multiple aptamers that bind to the biological markers on the EV surface, as well as exploring the size specifications of the aptamers. Meanwhile, the present invention uses EVs secreted by the cancer cells orginated from three different types of solid tumor cell lines, specifically colorectal cancer (HT29), breast cancer (SKBR3), and hepatocellular carcinoma (HepG2), to demonstrate the universality of the present method. Therefore, the sensitivity, simplicity of operation, and functionalization of the FluPADE detection determine that this method can be well applied to the research of a plurality of clinical oncology departments.

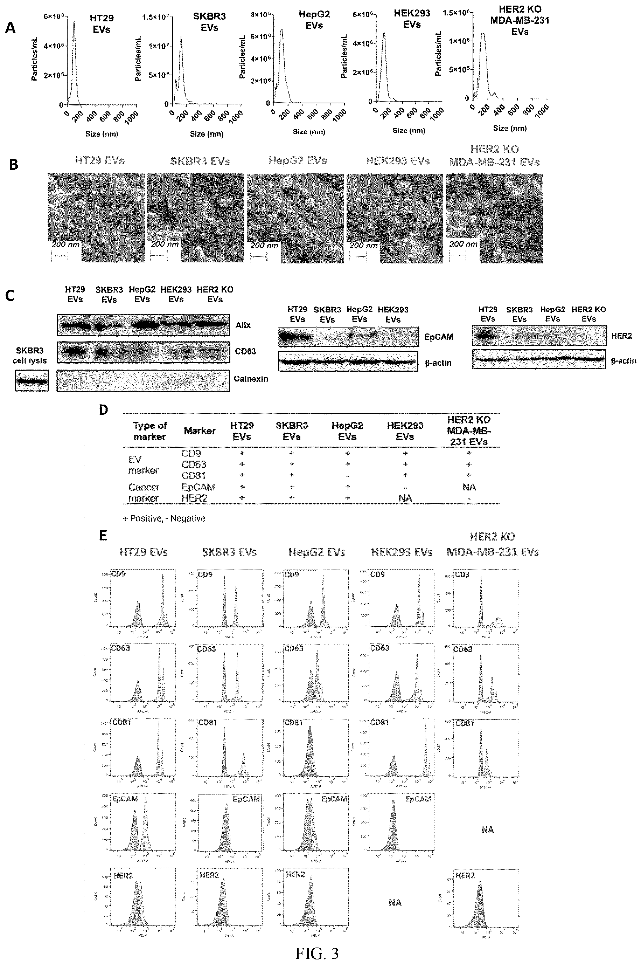

The present laboratory prepared extracellular vesicles from five different human cell lines, including HT29, SKBR3, HepG2, HEK293, and MDA-MB-231 cells , as well as the MDA-MB-231 cells with the HER2 gene knocked out, using standard ultrafiltration method (Centricon Plus-70 centrifugal filter device, MWCO 10kDa, Merck, Cat UFC701008). The particle size distribution of the EVs and their proportion in the total particle concentration were studied using the NTA technique. FIG. 3A shows the average size of the EVs from the five sources. Among these, the EVs sourced from the MDA-MB-231 line with the HER2 gene knock out had the largest size (142.3±10.3nm), while those from the HT29 cell line were the smallest (98.7±8.5nm). The main sizes of all five EVs fit within the size range of small EVs (size <200nm).

Furthermore, the surface morphology and shape of the EVs from the five different sources were characterized using scanning electron microscopy. The EVs from all five sources were spherical in shape (FIG. 3B). While the sizes of the EVs from the five sources were polydisperse, the majority of them had diameters less than 200nm. Interestingly, the EVs sourced from the MDA-MB-231 line with the HER2 gene knock out had the largest average size, keep consistent with the size distribution results determined by the NTA technique (FIG. 3A).

In some preferred embodiments, the EV markers include at least one of CD9, CD63, and CD81.

In some preferred embodiments, the cancer cell markers include EpCAM and/or HER2.

Referring to regulations of extracellular vesicles from the international association, EVs should contain at least one transmembrane protein and one cytoplasmic protein, and should not contain any negative regulatory proteins. Therefore, the present application demonstrates the presence of CD63 (a transmembrane protein) and Alix (a cytoplasmic protein) in EV by immunoblot assay and the absence of negative regulatory protein (calnexin, endoplasmic reticulum marker) by other cancer markers (EpCAM and HER2). As shown in FIG. 3C, CD63 and Alix were present in all EVs, while calnexin was not present in any of the five EVs. With regards to the cancer markers, EpCAM was detected in EVs sourced from HT29, SKBR3, and HepG2, but was not present in EVs sourced from HEK293. Importantly, HER2 gene expression was only detected in EVs sourced from HT29, SKBR3, and HepG2, but not in the EVs from the MDA-MB-231 with the HER2 gene knock out (FIG. 3C).

The surface proteins of EVs are the direct binding targets in the present invention. Therefore, besides characterizing proteins through blotting, the present invention also uses magnetic bead flow cytometry to characterize several biomarkers for five sources of EV, including three EV marker proteins (CD9, CD63, and CD81) and two cancer cell markers (EpCAM and HER2). Firstly, 10µm streptavidin-coated magnetic beads (Merck, Cat No.: LSKMAGT02) were washed with 10 volumes of a binding buffer solution (0.22µm) filtered, composed of phosphate buffered saline with 5% BSA) and then blocked with the same volume of the binding buffer solution at room temperature for 1 hour. Discarding supernatant, the blocked magnetic beads were resuspended in the binding buffer solution for EV separation. Then, 0.5µg of biotinylated antibodies is mixed with 2.5µg of blocked streptavidin-coated magnetic beads and they are incubated with gentle mixing at room temperature for 30 minutes. Antibody-coated magnetic beads were washed three times with ample washing buffer solution (0.22µm) filtered, composed of phosphate-buffered solution with 0.1 % Tween 20), followed by the addition of 100µL of 5×10⁸ EVs and 1 00µL of the binding buffer solution for overnight incubation at 4ºC. Finally, EV-magnetic bead complex was washed three times with the ample washing buffer solution.

In the flow cytometer, 2µL of 5µg EV-magnetic bead complex was cultured after mixing with 100µL of a binding buffer solution with a concentration of 50nM for the following antibodies. APC-anti-human CD9 (Thermo Fisher Scientific, Cat No.: A15698) or PE-conjugated anti-human CD9 antibody (BioLegend, Cat No.: 312106) was used to detect the mixture of EVs captured with anti-CD63 antibody (BioLegend, Cat No.: 353017) and anti-CD81 antibody (BioLegend, Cat No.: 349514). APC-anti-human CD63 (Thermo Fisher Scientific, Cat No.: A15712) or fluorescently labeled anti-human CD63 antibody (BioLegend, Cat No.: 353006) was used to detect the mixture of EVs captured with anti-CD9 antibody (BioLegend, Cat No.: 312112) and anti-CD81 antibody (BioLegend, Cat No.: 349514). Antibodies at the same concentration (50nM), APC-anti-human CD81 (Thermo Fisher Scientific, Cat No.: 17-0819-42) or fluorescently labeled anti-human CD81 antibody (BioLegend, Cat No.: 349504) was used to detect the mixture of EVs captured with anti-CD9 antibody (BioLegend, Cat No.: 312112) and anti-CD63 antibody (BioLegend, Cat No.: 353017). Alexa Fluor647^{®}-anti-human EpCAM antibody (50nM, R&D Systems, Cat No.: FAB9601 R100UG) was used to detect EVs captured with the anti-CD9 antibody (BioLegend, Cat No.: 312112), anti-CD63 antibody (BioLegend, Cat No.: 353017), and anti-CD81 antibody (BioLegend, Cat No.: 349514). PE-anti-human HER2 antibody (50nM, BioLegend, Cat No.: 324405) was used to detect EVs captured with the anti-CD9 antibody (BioLegend, Cat No.: 312112), anti-CD63 antibody (BioLegend, Cat No.: 353017), and anti-CD81 antibody (BioLegend, Cat No.: 349514). Tubes containing the antibodies and magnetic beads with bound EVs were placed in a HulaMixer sample mixer (Thermo Fisher Scientific, Cat No: 15920D) at room temperature for 30 minutes. Before analysis by flow cytometry (analyzing 10,000 samples in each test), the EV-coated magnetic beads were washed three times on a magnetic stand with an ample amount of washing buffer. The corresponding Median Fluorescence Intensity (MFI) and fluorescent histograms were recorded with a BD FACS-Canto^{™} II flow cytometer and then analyzed with FlowJo^{™} (v10.6.2).

FIG. 3D summarizes the profile of surface biomarkers of EVs sourced from 5 different cell origins. FIG. 3E shows histograms of the expression of surface marker proteins of EVs detected and analyzed by flow cytometry. As shown in the figure, EVs from all the origins exhibit the expression of marker proteins (CD9, CD63, and CD81), except for the HepG2-sourced EVs which lacked CD81 expression. Regarding cancer cell markers, EVs from HT29, HepG2, and SKBR3 show EpCAM expression, while EVs from HEK293 do not show EpCAM expression. These results detected and analyzed by the flow cytometry (FIG. 3D, FIG. 3E) are entirely consistent with the results of the immunoblotting analysis (FIG. 3C). Likewise, EVs sourced from HT29, SKBR3, and HepG2 exhibit HER2 protein expression, but it is not apparent in HER2 gene - knockout MDA-MB-231 sourced EVs. The present application, originally initiates the study of the (nucleic acid) aptamer-based fluorescence polarization analysis method based on these EV morphology with good characteristics, biochemical and cell biological characteristics.

The aim of this invention is to develop a test method capable of selectively and sensitively detecting cancer-sourced EVs. Thus, we used two different antibodies simultaneously in a test, capturing EVs through their interaction with EV markers or cancer cell markers. For example, biotinylated anti-human EpCAM antibody was used to capture HT29-sourced EVs, while biotinylated anti-human CD9/CD81 antibody was used to immobilize SKBR3-sourced EVs on a 96-well plate coated with streptavidin. Initially, the present invention demonstrated that the biotinylated antibodies and the streptavidin-coated microwell plates can indeed ensure the separation of EVs.

In some preferred embodiments, the said step S1 is carried out in a microwell plate, specifically using a streptavidin-coated microwell capture antibody, followed by the immobilization of EVs.

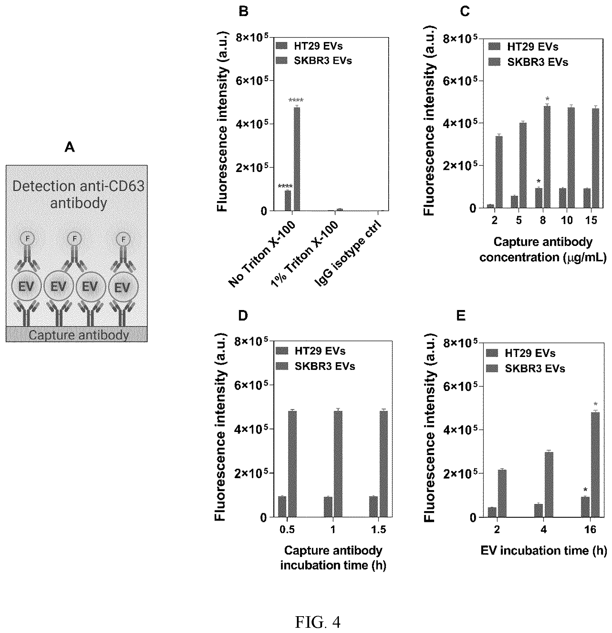

The fluorescence-labeled anti-human CD63 antibody was used to detect captured cancer EVs (FIG. 4A). FIG. 4B shows the fluorescence intensity (FI) of the anti-human CD63 antibody of EVs sourced from HT29 and SKBR3, either treated or untreated with Triton X-100. The immobilized EVs were treated with 1% Triton X-100 at room temperature for 30 minutes to lyse all vesicles. In samples that were not pretreated with Triton X-100, the CD63 signal of EVs captured by the anti-EpCAM antibody was significantly higher (p<0.0001) than the samples treated with Triton X-100, and significantly higher than the isotype-matched negative control anti-IgG antibody (BioLegend, Cat No.: 400104) coated plates, where no EVs were captured. These data validate the feasibility and specificity of capturing EVs released from cancer cells.

In some preferred embodiments, in the said step S1, when EVs sourced from HT29, the said antibody is biotinylated anti-human EpCAM antibody, and the said antibody concentration ranges from 2.0-15.0µg/mL; more preferably 8.0µg/mL.

In some preferred embodiments, in the said step S1, when EVs are sourced from SKBR3, the said antibody is biotinylated anti-human CD9/CD81 antibody (mass ratio 1:1), and the antibody concentration ranges from 2.0-15.0µg/mL; more preferably 8.0µg/mL.

As shown in FIG. 4C, in both cases, the CD63 signal increases within the range of 2.0µg/mL to 8.0µg/mL of antibody usage. When the antibody concentration exceeds 8.0µg/mL, there is no significant change in the CD63 signal. Thus, the optimal concentration for capturing EVs using biotinylated anti-EpCAM antibody or biotinylated CD9/CD81 antibody is 8.0µg/mL.

Further preferably, the optimal time for the capture antibody is between 0.1-1.5 hours; the immobilizing time is between 4-20 hours, and the immobilizing temperature is 4°C.

As shown in FIG. 4D, it determines the optimal time required for the streptavidin-coated microwells capture antibody. When the capture antibody is cultured in the streptavidin-coated microwells for 0.5 hours, 1 hour, and 1.5 hours, there is no significant difference in the signals detected from the antibodies of the two EV sources. It indicates that a culture time of 0.5 hours is sufficient. In summary, after immobilizing, the optimal time for culturing EVs with the captured antibody is 16 hours (FIG. 4E).

FP is a homogeneous technology that can accurately quantify ligand-target interactions; it should be noted that FP characterization results depend on the binding affinity during interaction. To compare the FP response differences when antibodies and aptamers interact with EVs, the present invention first determined the binding affinities of these ligands with the EVs used in this invention. All aptamers were initially synthesized through linear oligonucleotides and folded into appropriate 3D structures before use, and the folding schemes adopted were rigorously regulated and determined.

In some preferred embodiments, the said aptamer includes at least one of CD63-BP, HER2-HApt, and HER2-2A.

In some preferred embodiments, the said aptamer is folded before use, the specific steps include: diluting the aptamer to the target concentration using a phosphate buffer solution containing 0.5-2.0mM MgCl₂, then denaturing it at 90-98□ for 2-10 minutes, incubating on ice or at room temperature for 5-20 minutes, and then refolding at 35-38□ for 10-30 minutes.

Further preferably, for the CD63-BP aptamer and the negative control DNA aptamer, they are diluted to the target concentration with a phosphate buffer solution containing 1.0mM MgCl₂, then denatured at 95□ for 5 minutes, incubated on ice for 10 minutes, and then refolded at 37□for 15 minutes. For the HER2-HApt aptamer, it is diluted to the target concentration with a phosphate buffer solution containing 1.0mM MgCl₂, then denatured at 95□ for 5 minutes, incubated on ice for 15 minutes, and then refolded at 37□ for 15 minutes. For the HER2-2A aptamer, the aptamer is diluted with the phosphate buffer solution and added with 2.5mM MgCl₂, then denatured at 95□ for 5 minutes, incubated at room temperature for 10 minutes, and then refolded at 37□ for at least 15 minutes.

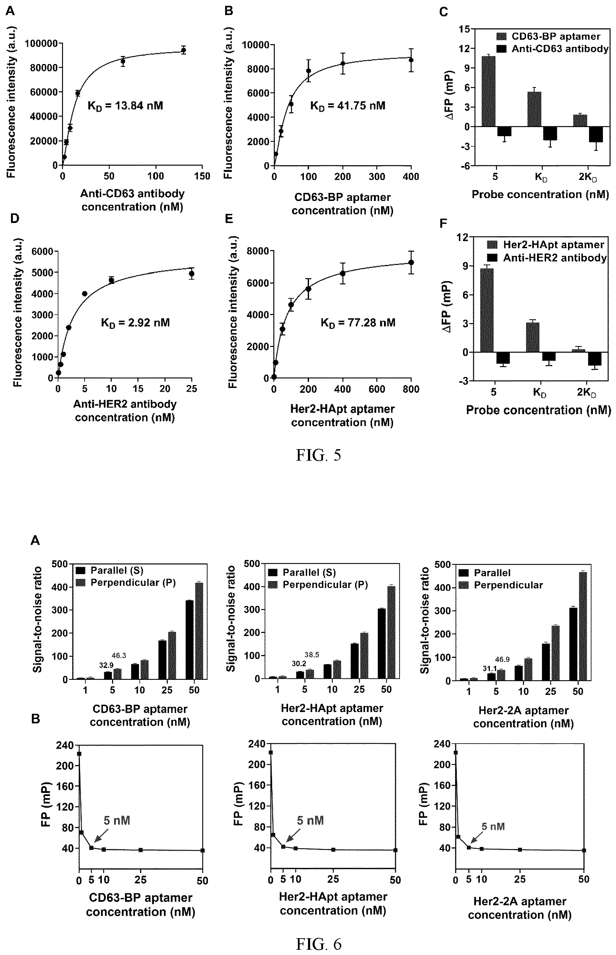

Interestingly, experimental results show that the binding affinity of antibodies is significantly higher than that of the corresponding aptamers (FIG. 5). Specifically, the apparent dissociation constants (K_{D}) of the anti-CD63 antibody and the CD63-BP aptamer (5'-fluorescently labeled CAC CCC ACC TCG CTC CCG TGA CAC TAA TGC TA-idT-3', Angew Chem Int Edit, 2017, 56, 11916-11920.) for EVs sourced from HT29 are 13.84nM and 41.74nM, respectively. The K_{D}s of the anti-HER2 antibody and the HER2-HApt aptamer (5'-fluorescently labeled GCA GCG GTG TGG GGG CAG CGG TGT GGG GGC AGC GGT GTG GGG-idT-3', Proc. Natl. Acad. Sci. U.S.A., 2013, 110, 8170-8175.) for EVs sourced from SKBR3 are 2.92nM and 77.28nM, respectively. Theoretically, in FP assays, the concentration of the probe substance should not exceed twice the binding affinity of the probe substance to its target, to avoid stoichiometry titration of the probe substance. Therefore, the present invention compared the FP responses under three different concentrations of probe substances, including concentrations equal to K_{D}, twice K_{D}, and 5nM. As shown in FIG. 5C and FIG. 5F, when using anti-CD63 and anti-HER2 antibodies as probe substances to detect immobilized EVs, all three concentrations showed negative changes of ΔFP values for samples with no EVs (only free ligands) and with EVs (partially bound ligands), indicating that these two antibodies could not be detected by FP. In sharp contrast, the fluorescently labeled CD63-BP aptamer and HER2-HApt aptamer showed positive ΔFP values at all three concentrations (Figs. 5C, 5F). Results show that the FP of the fluorescently labeled CD63-BP aptamer (ΔFP) decreases as the aptamer concentration goes from 5nM (ΔFP of 10.8±0.3mP) to 2K_{D} (1.8±0.2mP). Similarly, the ΔFP of the HER2-HApt aptamer decreases from 8.7±0.4mP at 5nM to 0.3±0.3mP at 2K_{D}. The decrease in ΔFP with increasing aptamer ligand concentration is likely due to the increase in the proportion of unbound ligands, which helps in the observed fluorescence depolarization. Therefore, the present application proves that in the detection of CD63 and HER2 aptamers at three different concentrations, immobilized EVs from HT29 and SKBR3 can be clearly detected by FP, but antibodies binding to the same target proteins on the EV surface cannot be detected.

Further preferably, the target concentration of the said aptamer is 1-8nM; even more preferably 5nM.

Before conducting the established FluPADE test, in order to determine the optimal concentration of the aptamers used, the present application investigated the signal-to-noise ratio (S/N) of the parallel and vertical fluorescence intensity of various concentrations of fluorescently labeled CD63-BP aptamer/HER2-HApt aptamer/HER2-2A aptamer (5'-fluorescently labeled TTT CCT CCA TTG G-inverted thymidine-3', #202111267773.2). As shown in FIG. 6, the signal-to-noise ratio of parallel and vertical fluorescence intensities increased with the increase in concentration of all three aptamers (FIG. 6A). The parallel/vertical fluorescence intensity ratio of CD63-BP aptamer increased from 6.9±0.7 or 7.6±3.7 at 1nM to 341.2±2.1 or 419.0±5.9 at 50nM, and for HER2-HApt aptamer from 8.6±0.8 or 10.6±2.1 at 1nM to 303.5±2.3 or 402.3±6.4 at 50nM. The range of parallel/vertical fluorescence intensity ratio for HER2-2A aptamer was from 8.8±0.6 or 11.4±1.9 at 1nM to 313.3±5.7 or 467.4±6.4 at 50nM. It's known to those skilled in the art that in FluPADE detection, the optimal concentration of the ligand should be as low as possible, where the contribution of the background parallel and vertical fluorescence intensities to the final FP is minimal, so the FP signal mainly comes from the aptamers and not from background noise. In the present invention, the signal-to-noise ratio of parallel and vertical fluorescence intensities for all three aptamers was higher than 30 at 5nM concentration (FIG. 6B). Based on these values, the parallel and vertical fluorescence intensities of the detected aptamers dominate in the final FP signal. Furthermore, the concentration of 5nM is much lower than the K_{D} of the aptamers, ensuring the reliability of the FP signal detection. Additionally, the FP signal remains unchanged when the concentration of the aptamers reaches 5nM and above (FIG. 6B). Therefore, the optimal concentration of the three fluorescently labeled nucleic acid aptamers is preferably 5nM.

In some preferred embodiments, the said step S2 specifically involves adding 60-140µL buffer solution containing fluorescently labeled nucleic acid aptamers to the product obtained in the step S1 and placing the microplate on a shaker to incubate in the dark at room temperature for 0.5-2 hours.

In some preferable embodiments, in said step S3, the fluorescent polarization signal is read by a multi-functional plate reader; the said multi-functional plate reader is equipped with an excitation filter of 475-490nm and an emission filter of 520-565nm.

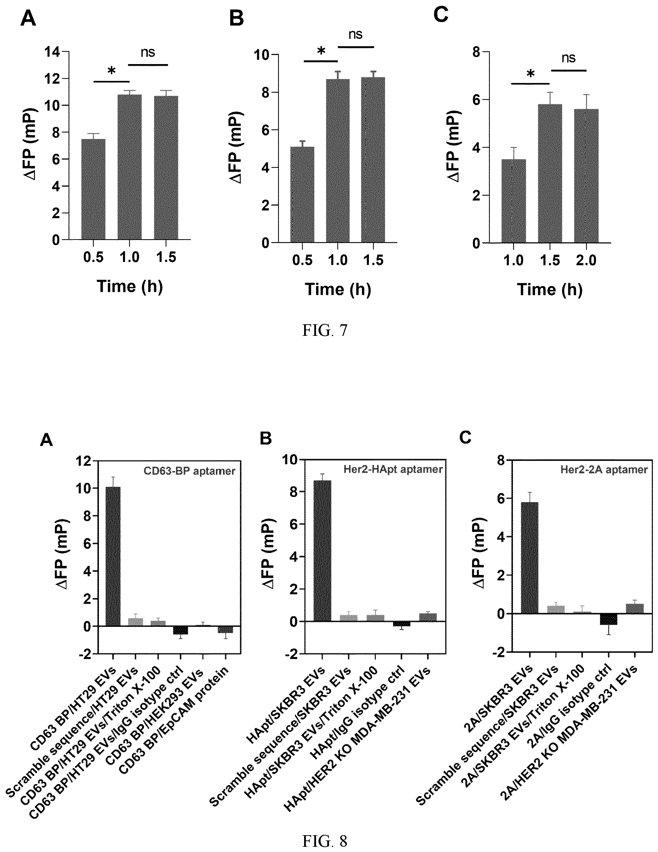

The present invention explored the cultivation time of three kinds of aptamers with immobilized EVs and optimized the FluPADE detection scheme. As shown in FIG. 7A, the ΔFP of CD63-BP aptamer increased significantly from 7.5±0.4mP at 0.5 hours to 10.8±0.3mP at 1.0 hour (p<0.05), and ΔFP did not increase further after 1.5 hours. Similarly, the best cultivation time for HER2-HApt aptamer is 1.0 hour, with a ΔFP peak value of 8.7±0.4mP (FIG. 7B). On the other hand, the HER2-2A aptamer reached a ΔFP peak value of 5.8±0.5mP at 1.5 hours (FIG. 7C).

Under conditions of 4°C, using 8µg/mL of capture antibody in a 96-well plate to fix the EVs for more than 16 hours, after washing, 100µL of one of the 5.0nM fluorescently labeled aptamers (CD63-BP aptamer: PBS with 1.0mM MgCl₂; HER2-HApt aptamer: PBS with 5.0mM MgCl₂; HER2-2A aptamer: PBS with 2.5mM MgCl₂) was added in the binding buffer solution. The microplate was placed on a shaker (Thermoline Scientific, Model: TL400) at room temperature in the dark for 1.5 hours of incubation. Finally, the FP signal was read by the multifunctional plate analyzer CLARIOstar Plus (BMG Labtech) using an excitation of 485nm filter and an emission filter of 535nm, to perform the measurement of the FP values. All FP values are expressed in millipolarization (mP) units, and the FP value of each sample is calculated from the average value of three different wells, and the FP value of each well is the average of three measurements. Control wells (containing only free aptamers) were prepared in the same way, except that only PBS was added to the wells.

To prove that the invention detects EVs, not soluble membrane fragments or free proteins, a control group was set up in the experiment. The immobilized EVs were treated at room temperature for 15 minutes with 1% Triton X-100 (polyethylene glycol octyl phenyl ether) to ensure no intact EVs remained. For the single-factor control samples of free EVs and proteins, 100µL of 50nM EpCAM protein was added to the wells, and EVs from cells not expressing EpCAM or HER2 were immobilized as a control group for further specificity control. They were treated for 15 minutes at room temperature to ensure no intact EV remains. For single factor control samples of free EV and protein, 100 µ L of 50nM EpCAM protein was added to the wells and EV of cells not expressing EpCAM or HER2 was immobilized as a control group for further specificity control.

In some preferred embodiments, the said buffer includes synthetic buffer or human plasma.

Further preferably, the said synthetic buffer solution is a PBS buffer solution.

Further preferably, the said human plasma is sourced from donors of any blood type, including type A, B, AB, O, Rh+, or Rh-;
further preferred, the said human plasma comes from donors aged 0-120 years;
further preferred, the said human plasma is sourced from either healthy individuals or non-healthy individuals; further preferably, the said human plasma is from non-healthy individuals, preferably cancer patients.

In liquid biopsies, the EVs to be tested are suspended in human plasma rather than PBS. Based on this, to simulate liquid biopsy conditions, EVs derived from cell lines are added to human plasma. For this purpose, before separating the EVs, cell line-sourced EVs are added to human plasma at a 1:10 (v/v) ratio, using an 8µg/mL required antibody to capture the EVs from cell lines present in the plasma. The control well is treated similarly, with the difference being that the EVs sourced from the cell line are replaced with the same volume of PBS.

As shown in FIG. 8A, except for using the anti-IgG antibody as an isotype-matched negative control, an antibody suitable for EpCAM was used to fix the EVs sourced from HT29. In this experiment, a fluorescently labeled CD63-BP aptamer was used to detect the presence of the captured EVs. It was found that the ΔFP (fluorescence polarization change) of the negative control aptamer was significantly lower than that of the HT29-sourced EVs (ΔFP is 10.1±0.7mP, p<0.0001). The said negative control aptamer includes a random nucleotide sequence that doesn't bind with CD63, Triton X-100 that dissolves EVs, and EVs sourced from HEK293 that don't express EpCAM. In fact, the ΔFP levels of these negative controls were below the detection limit of this experimental method. Additionally, the ΔFP for the samples of EVs captured with the isotype-matched control antibody was -0.6±0.3mP, consistent with the aforementioned results, further proving the selectivity of this method for EVs sourced from HT29. To rule out the possibility that ΔFP was caused by the binding of the CD63 aptamer and the free EpCAM protein captured by the anti-EpCAM antibody, a further negative control was set up in the present invention, where only free EpCAM protein was added to wells coated with anti-EpCAM, without adding EpCAM-positive EVs. As shown in FIG. 8A, the ΔFP of this negative control was -0.5±0.4mP, indicating that the positive ΔFP was not caused by the interaction of the fluorescently labeled CD63-BP aptamer with the free EpCAM protein immobilized in the micro-wells. In summary, FIG. 8A clearly demonstrates that this experiment has selectivity and specificity on EpCAM-positive EVs sourced from HT29, and the interactions between the fluorescence, EVs sourced from HT29, and antibody coating have a negligible impact on the FP signal.

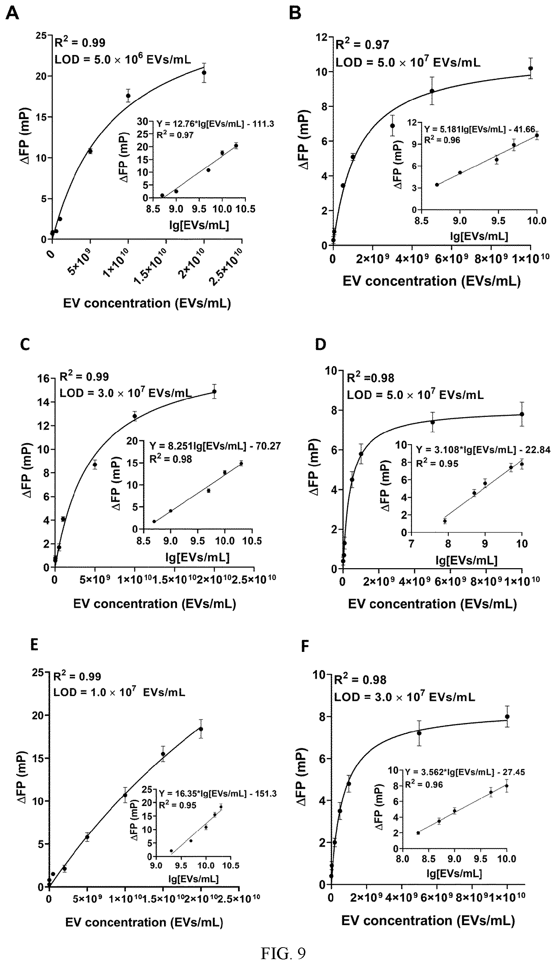

Furthermore, the present invention detected the specificity of the FP signal of the fluorescently labeled HER2 DNA aptamer to EVs captured in a micro-well plate by the anti-CD9 and CD81 antibodies. In a similar mode, the ΔFP of the HER2-HApt and HER2-2A aptamers originating from HER2 positive EVs (respectively 8.7±0.4mP and 5.8±0.5mP) was significantly higher than the ΔFP of the negative control samples. The negative control samples included a random nucleotide sequence aptamer, an EV-dissolving detergent, an isotype-matched control antibody coating, and EVs sourced from MDA-MB-231 cells with the HER2 gene knocked out (p<0.0001, FIG. 8B, FIG. 8C). Indeed, the ΔFP levels of these negative control samples were also below the detection limit of the present experimental method (FIG. 9).

In the present invention, EVs from cancer cell lines, prepared in a wide concentration range of mixtures with a buffer solution(F-PBS, phosphate buffer solution filtered through a 0.2-micron membrane) or human plasma, were used to determine the limit of detection (LOD) and linear dynamic range (LDR) of the experimental method.

The LOD and LDR for EVs in F-PBS or human plasma detected by each aptamer in FluPADE are shown in FIG. 9. Interestingly, unlike the detection values and linear dynamic range for EVs diluted in PBS (compare FIGs. 9A, C, E with FIGs. 9B, D, F), FluPADE tests using human plasma EVs showed increased LOD and decreased LDR. For the FluPADE test based on the CD63-BP aptamer, the LOD of EV increased from 5.0×10⁶ HT29 EVs/mL in PBS to 5.0×10⁷ HT29 EVs/mL in human plasma diluted EVs. The LDR of total EV in PBS is 5.0×10⁸-2.0×10¹⁰ HT29 EVs/mL, which is broader than the LDR of EVs in human plasma (5.0×10⁸-1.0×10¹⁰) (FIG. 9A and FIG. 9B). Similarly, the FluPADE results for the HER2-HApt aptamer showed that the LOD for SKBR3 sourced EVs in PBS was lower at 1.0×10⁷ EVs/mL; whereas, the LOD in human plasma was 3.0×10⁷ SKBR3 EVs/mL. The test results also showed a downward shift in the LDR for EVs in PBS, specifically 2.0×10⁹-2.0×10¹⁰ SKBR3 EVs/mL; whereas, the LDR for EVs in human plasma was broader, specifically 2.0×10⁸-1.0×10¹⁰ SKBR3 EVs/mL (FIG. 9D and FIG. 9F). The FluPADE detection in the present invention targeting HER2 in SKBR3 sourced EVs in both PBS and human plasma, showed that the LOD measured using the HER2-2A aptamer was lower compared to using the HER2-HApt aptamer, but the former's LDR was narrower than the LDR of the HER2-HApt aptamer (FIGs. 9C, E vs. FIGs. 9D, F).

In the experiment, the same detection aptamers were used to analyze allogenic EVs in PBS or human plasma. It was found that the differences in LODs and LDRs were determined by the variance between the total amount of available biomarker proteins in EVs sourced from cell lines in PBS and those in human plasma. The volume of EVs affixed by the capturing antibodies in the microwells and the positive biomarker EVs in PBS or blood plasma were reasons leading to the differences in the final output of the fluorescence polarization signal. Therefore, the present invention explored the contribution of EVs biomarker protein amounts in human plasma.

In short, the above described method of using antibodies to isolate EVs from human cell lines, was adopted to separate EVs originating from human plasma. Subsequently, 100µL of 50nM antibodies was used to culture the immobilized EVs from human plasma. These antibodies included fluorescently labeled anti-EpCAM antibodies (the same as those used during EV capture), fluorescently labeled anti-CD63 antibodies (used during EV immobilizing), PE-conjugated anti-CD9 antibodies (used for EV capture), fluorescently labeled anti-CD81 antibodies (used for EV capture), and fluorescently labeled anti-HER2 antibodies (anti-CD9/CD81 antibodies used for EV capture). After incubation at room temperature for 30 minutes, it was washed three times with 200µL of 0.1% F-TPBS. The fluorescence intensity spectrum within the wavelength range of 520nm to 660nm was then recorded using the CLARIOstar Plus (BMG Labtech) multifunction plate reader.

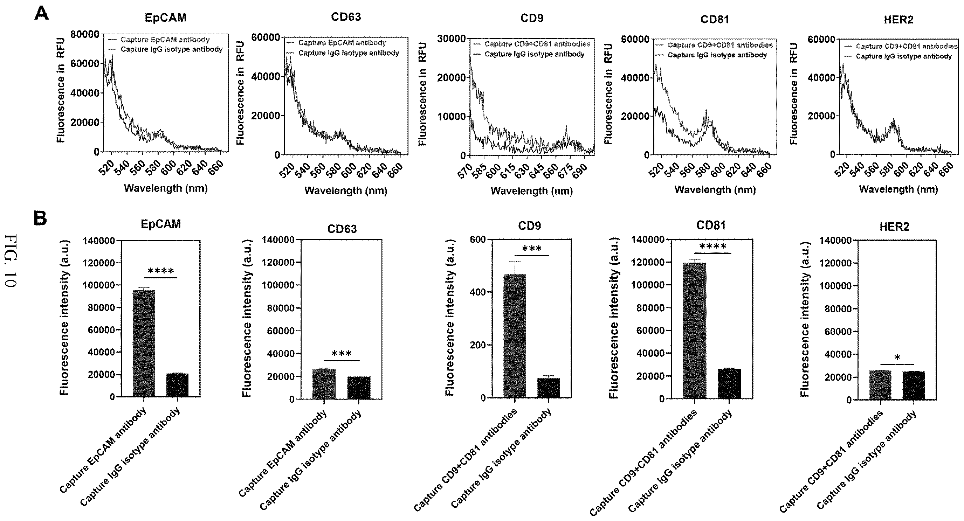

As shown in FIG. 10, EVs isolated from human plasma using anti-EpCAM antibodies clearly showed the expression of cancer marker proteins, and EV marker protein CD63 was also distinctly detected in the EVs separated from human plasma. The levels of EpCAM and/or CD63 proteins in EVs separated from human plasma could have an impact on the following aspects: the performance of the fluPADE detection method based on the CD63-BP aptamer for detecting EVs isolated from cancer cell lines. Firstly, the presence of EpCAM in human plasma EVs might lead to a reduction in HT29-sourced EVs. This scenario arises during the competitive binding process between the anti-EpCAM antibody captured in human plasma and the EpCAM protein in the plasma EVs as well as allogeneic proteins in HT29-sourced EVs. This is supported by the stronger EpCAM signal from HT29-sourced EVs in F-PBS. Differences in the output EV signals during the study indicated that the amount of anti-EpCAM antibodies immobilized on the microwells is limited. The number of binding sites in the microwells, along with the competitive binding between human plasma EVs and HT29-sourced EVs, led to saturation of the available binding sites of the anti-EpCAM antibody. As a result, the number of HT29 separated from human plasma was significantly less compared to the large number of HT29-sourced EVs separated from PBS. Secondly, although the CD63 expression level of EVs in human plasmais is much lower than in HT29-sourced EVs (fluorescence intensity 6206.5 corresponds to human plasma EVs of 9.2×10⁸, and fluorescence intensity 84345.1 corresponds to HT29-sourced EVs of 2.0×10⁸), the interaction between the CD63-BP aptamer and CD63 protein in human plasma EVs might promote the overall FP signal detection. The synergy of these effects would lead to the apparent LOD of HT29-sourced EVs bound to human plasma rising from 5×10⁶ to 5×10⁷ EVs/mL, with the LDR range dropping from 5.0×10⁸-2.0×10¹⁰ to 5.0×10⁸-1,0×10¹⁰ EVs/mL. Similarly, the experiment found that CD9, CD81, and HER2 exist in human plasma EVs separated by anti-CD9/CD81 antibodies (FIG. 10). Similarly, the presence of this class of EV biological marker proteins in human plasma EVs provides an explanation for the observed FP performance changes between SKBR3-sourced EVs suspended in PBS or human plasma.

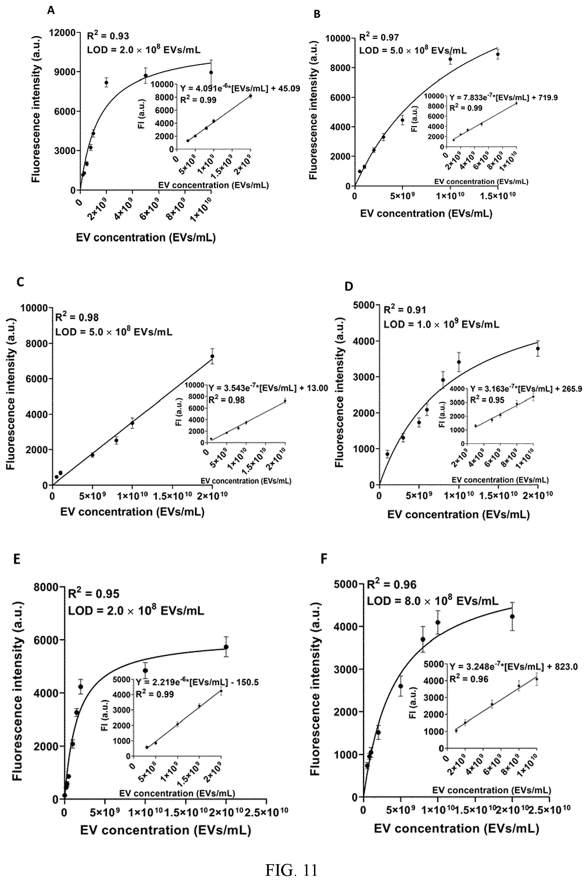

Fluorescence intensity is the most commonly used detection modes in fluorescence detection, and fluorescence polarization is rarely used as a detection modes, especially in the field of medical diagnostics. As shown in FIG. 11, the present invention employs the same EV immobilization method and fluorescently labeled aptamer to compare EV performance based on fluorescence intensity and fluorescence polarization pattern, particularly presented by LOD and LDR.

For detection of EVs in PBS based on fluorescence intensity, different concentrations of HT-29-sourced EVs and SKBR3-sourced EVs were immobilized in wells by a mixture of anti-EpCAM antibody or anti-CD9/CD81 antibody at 4□ for 16 hours and detected with CD63 or HER2, respectively. After washing, 100µL of a buffer solution containing one of the following aptamers was added: 800nM fluorescently labeled CD63-BP aptamer, 800nM fluorescently labeled HER2-HApt aptamer, or 800nM fluorescently labeled CD63-BP aptamer. Then, 100µL PBS containing 50nM fluorescently labeled CD63 antibody or 50nM fluorescently labeled HER2 antibody was added. The immobilized EV and the probe substance were incubated for 1 hour at room temperature in the dark on a shaker (Thermoline Scientific, Model No.: TL400). After three times washing with 200µL of the wash buffer solution, fluorescence intensity was measured with a multifunctional disc analyzer, CLARIOstar Plus (BMG Labtech) at an excitation filter of 485nm and an emission filter of 535nm. Control wells with background fluorescence were prepared in the method as described above and biotin-labeled IgG isotype-matched control antibodies were used to replace the biotin-labeled EV marker-specific antibodies.

For detection of EV from cell lines in human plasma based on fluorescence intensity, the required number of EVs was added to human plasma at a ratio of 1:10 (v/v) before immobilizing the EV. EV in human plasma was then detected in the same manner as that in PBS.

FIG. 11 shows the relationship between fluorescence intensity and EV concentration, LOD and LDR, in particular the fluorescence intensity was measured for HT29-sourced EVs or SKBR3-sourced EVs from F-PBS (FIGs. 11A, C, E) or human plasma (FIGs. 11B, D, F) using the CD63-BP aptamer, HER2-HApt aptamer or HER2-2A aptamer. The LOD for the fluorescence intensity detection of cancer cell-secreted EVs in F-PBS using fluorescently labeled CD63-BP aptamer is 2.0×10⁸ HT29 EVs/mL (FIG.11A). When using a fluorescently labeled HER2-HApt aptamer for fluorescence intensity detection of SKBR3-sourced EV, the LOD thereof is 5.0×10⁸ SKBR3 EVs/mL (FIG.11C). The LOD for fluorescence intensity detection with fluorescently labeled HER2-2A aptamer is 2.0×10⁸ SKBR3 EVs/mL (FIG.11E).

The present invention also determined the LODs of fluorescence intensity of EVs in human plasma. The LOD of fluorescence intensity measured by the CD63-BP aptamer in human plasma increased by approximately two and a half times, specifically to 5.0×10⁸ HT29 EVs/mL. The LOD of the HER2-HApt aptamer increased by twice, specifically to 1.0×10⁹, and the LOD of the HER2-2A aptamer increased by four times, specifically to 8.0×10⁸ SKBR3 EVs/mL (FIG. 11D, FIG. 11F). The significant correlation difference in LOD between EVs in PBS and EVs in human plasma may be related to biomarkers in EVs in human plasma. Interestingly, the LOD of FluPADE detection is much lower than that of fluorescence intensity detection using the same detection aptamer. Specifically, the LOD of FluPADE detection using the CD63-BP aptamer in PBS and human plasma is respectively four times (respectively 5.0×10⁶ EVs/mL and 2.0× 10⁸ HT29 EVs/mL) and ten times (respectively 5.0×10⁷ EVs/mL and 5.0×10⁸ HT29 EVs/mL) lower than that of fluorescence intensity detection. Similarly, FP detection using the HER2-HApt aptamer in PBS and human plasma is approximately seventeen to twenty-six times more sensitive than fluorescence intensity detection. Specifically, the LOD of the HER2-HApt aptamer in PBS is 3.0×10⁷ SKBR3 EVs/mL, the LOD of the HER2-2Aaptamer is 1,0×10⁷ SKBR3 EVs/mL, the LOD of the HER2-HApt aptamer in human plasma is 5.0×10⁷ SKBR3 EVs/mL, and the LOD of the HER2-2A aptamer is 3.0×10⁷ SKBR3 EVs/mL.

In addition to the increased lower limit and decreased upper limit of LDR for fluorescence intensity detection based on the HER2-HApt aptamer (FIG. 11), both the lower and upper limits of LDR for EV fluorescence intensity in human plasma both show an upward trend. Specifically, in PBS and human plasma, the fluorescence intensity LDR of the fluorescently labeled CD63-BP aptamer is 3.0× 10⁸-2.0×10⁹ EVs/mL, and the fluorescence intensity LDR of the HER2-2A aptamer is 1.0×10⁹-1.0× 10¹⁰ EVs/mL. It can be clearly seen from these results that the lower limits of fluorescence intensity LDR of the three aptamers in human plasma are all higher than the LDR lower limits in PBS. This upward shift trend in LDR may be due to the additional competitive binding of biomarkers on EVs in human plasma during EV immobilizing and detection. In contrast, FluPADE assays based on aptamers, especially when detecting HER2-positive EVs, can detect cancer EVs in PBS and human plasma at lower concentrations and quantify EVs over a wider EV concentration range, whereas fluorescence intensity detection based on aptamers cannot achieve such an effect.

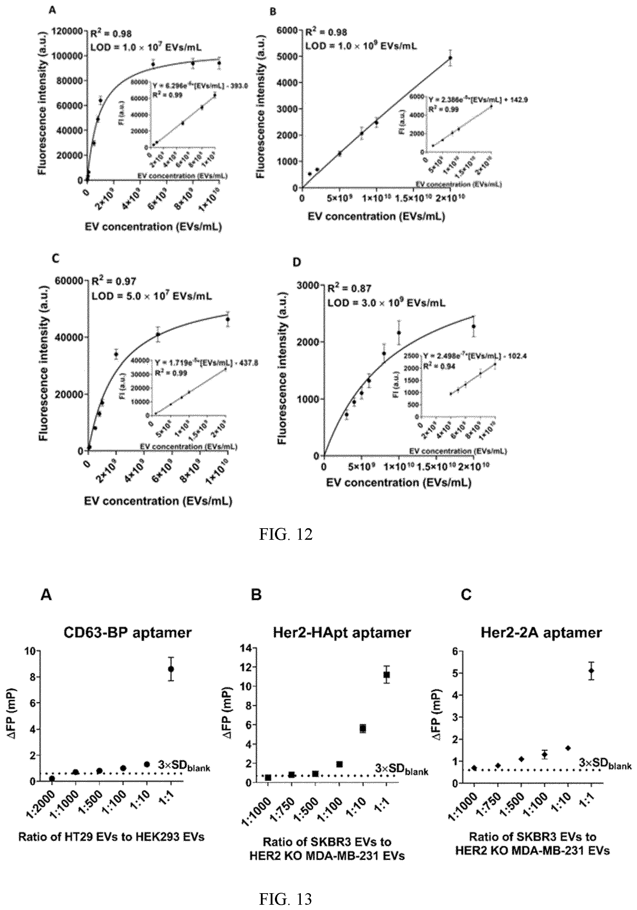

Based on exploring the performance of the fluorescence intensity test method using aptamers, although antibodies cannot effectively perform FP detection of EVs, the present application also explored the detection performance of fluorescence intensity using antibodies. FIG. 12 shows the relationship between fluorescence intensity based on antibody testing and EV concentration, LOD, and LDR. For EV detection in PBS, the LOD of anti-CD63 antibody detection FI is 1.0× 10⁷ HT29 EVs/mL, and the LDR is 5.0×10⁷-1.0× 10⁹ HT29 EVs/mL (FIG. 12A); the LOD of anti-HER2 antibody detection Fl is 1.0×10⁹ SKBR3 EVs/mL, and the LDR is 2.0×10⁹-2.0×10¹⁰ SKBR3 EVs/mL (FIG. 12C). For EV detection in human plasma, the LOD of anti-CD63 antibody detection Fl is 1.0×10⁷ HT29 EVs/mL, and the LDR is 5.0×10⁷-1.0×10⁹ HT29 EVs/mL(FIG. 12A); the LOD of anti-HER2 antibody detection Fl is 5.0×10⁷ HT29 EVs/mL, and the LDR is 1.0v10⁸-2.0× 10⁹ HT29 EVs/mL (FIG. 12B); the LOD of anti-HER2 antibody detection FI is 3.0×10⁹ SKBR3 EVs/mL, and the LDR is 5.0×10⁹-1.0× 10¹⁰ SKBR3 EVs/mL (FIG. 12D).

These results strongly demonstrate that the FluPADE provided by the present invention is superior to traditional fluorescence intensity-based detection methods in detecting cancer-sourced EVs in buffer solutions and human plasma, with higher sensitivity and a broader dynamic range (Table 1).

In some preferred embodiments, when the aptamer is CD63-BP, the said detection method has an LOD of ≤5×10⁷ EVs/mL, and an LDR 5×10⁸-2×10¹⁰ EVs/mL. When the aptamer is HER2-HApt, the said detection method has an LOD of ≤5×10⁷ EVs/mL, and an LDR 8×10⁷-2×10¹⁰ EVs/mL. When the aptamer is HER2-2A, the detection method has an LOD of ≤3×10⁷ EVs/mL, and an LDR 2×108-2×10¹⁰EVs/mL.

One of the key challenges for EV-based cancer diagnosis is the low abundance of cancer-sourced EVs in samples. This is because the cancer-sourced EVs are released into an EV reservoir, which contains EVs released from about 200 types of healthy human cells in the biological fluids. This situation makes identifying cancer cell-derived EVs equivalent to finding a needle in a large sea. To determine the sensitivity of the CD63-BP aptamer-based FluPADE detection, the present invention continously titrated EpCAM positive EVs sourced from HT29 cells into EpCAM negative EVs sourced from HEK293 cells at ratios of 1:2000, 1:1000, 1:500, 1:100, and 1:10, maintaining the total EV concentration at 1.0005×10¹⁰ EVs/mL.

For FluPADE detection using either HER2-HApt or HER2-2A aptamer, HER2 positive EVs from SKBR3 cells were continously titrated into EVs from HER2 gene-knockout MDA-MB-231 cells at ratios of 1:1000, 1:750, 1:500, 1:100, and 1:10. The total EV concentration was respectively set at 3.0030×10¹⁰ EVs/mL (for HER2-Hapt aptamer) and 1.0010×10¹⁰ EVs/mL (for HER2-2A aptamer).

For FP-based EV detection in PBS, 8µg/mL of anti-EpCAM antibody for HT29-sourced EVs or anti-CD9/CD81 antibody for SKBR3-sourced EVs was added to streptavidin-coated microwells. After incubating at room temperature for 30 minutes and washing, the EVs were immobilized for over 16 hours at 4□. 100µL of PBS containing one of the following aptamers (5nM) was added in each of the microwells: fluorescently-labeled CD63-BP, fluorescently-labeled HER2-HApt, or fluorescently-labeled HER2A. They were then incubated on a shaker (Thermoline Scientific, model: TL400) in a light-protected room-temperature environment. The first two aptamers were incubated for 1 hour, and the third for 1.5 hours.

Finally, the FP signal was measured on the multifunctional plate analyzer CARIOstar Plus (BMG Labtech), with an excitation filter at 485nm and an emission filter at 535nm. Control wells for free ligand FP were prepared similarly, except without the addition of EVs. Notably, the total concentration of EVs needs to be maintained at 10¹⁰ EVs/mL, to simulate physiological concentration of EV in body circulation. This ensures the results of the present invention are clinically relevant and can be applied to pathological experiments. The sensitivity of the tests in the present invention is defined by the minimum ratio where the detected FP signal is equal to or greater than the FP signal at the LOD.

As shown in FIG. 13, with a total EV concentration is kept constant, ΔFP increases as the ratio of labeled positive EVs to labeled negative EVs increases. Using the CD63-BP aptamer to detect EVs, the ΔFP at a 1:2000 ratio was found to be below the ΔFP of 3×SD_{blank} (respectively 0.2±0.1mP and 0.6±0.1mP). However, the ΔFP of this experiment significantly increased from 0.7±0.1 at a 1:1000 ratio to 8.6±0.9mP at a 1:1 ratio. This result indicates that the FluPADE detection using CD63-BP aptamer not only detects at the LOD level (5×10⁶ EVs/mL) but can also detect EpCAM positive EVs sourced from HT29 even when at the condition of one thousand times EpCAM negative EVs.

As shown in FIG. 13, the sensitivity of FluPADE detection based on the HER2-HApt aptamer is to detect at least one HER2 positive EV against a background of 750 HER-2 negative EVs(FIG. 13, HER2-HApt aptamer). In contrast, the sensitivity of detection using the HER2-2A aptamer is to detect at least one HER2 positive EV against a background of 1000 HER2 negative EVs. Detection based on the HER2-HApt aptamer showed higher sensitivity.

A mature method for detecting cancer-sourced EVs through liquid biopsy not only requires high sensitivity but should also be capable of distinguishing EVs from different sources using the same biomarkers. EV released by tumors at the primary site and EV released from metastatic sites represent different entities in terms of genetics and phenotype. Even EVs released by cancer cells from the same patient may qualitatively express preferred biomarkers, , it can be envisioned that the abundance of surface biomarker proteins on EVs released from tumors at different primary sites, different growth stages, or different clinical courses may vary quantitatively. From a diagnostic perspective, the FluPADE provided by this invention is very beneficial for detecting different EV populations from various cancer cells based on the quantitative differences of abundance in surface biomarker. To demonstrate the capability of FluPADE in detecting and/or differentiating EVs from different sources, the present invention established a model system wherein EVs come from three cell lines of three common solid cancers. Initially, the differences in the abundance of CD63 and HER2 in EVs prepared from colorectal cancer (HT29), breast cancer (SKBR3), and hepatocellular carcinoma (HepG2) were determined in the present invention. The method for measuring the abundance of CD63 and HER2 in these EVs is similar to the aforementioned method, with the distinction of using 50nM of Alexa Fluor 647^{®}-conjugated anti-HER2 antibody (BioLegend, Cat No.: 324412) to quantify HER2-positive EVs.

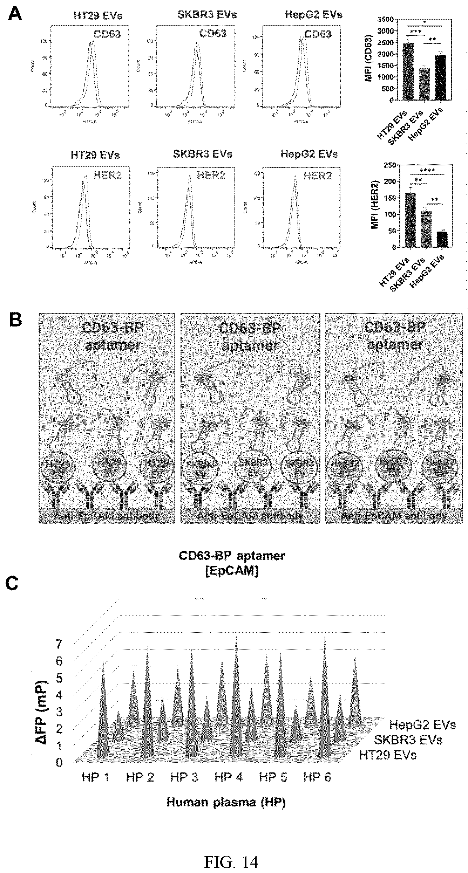

As shown in FIG. 14A, EVs from different cancer origins dshow varying amounts of surface marker proteins for EV biomarkers (CD63) and cancer biomarkers (HER2). The differences in the abundance of EV surface marker proteins will lead to variations in the degree of fluorescence polarization generated when detecting fluorescently labeled aptamers binding to the EV surface.

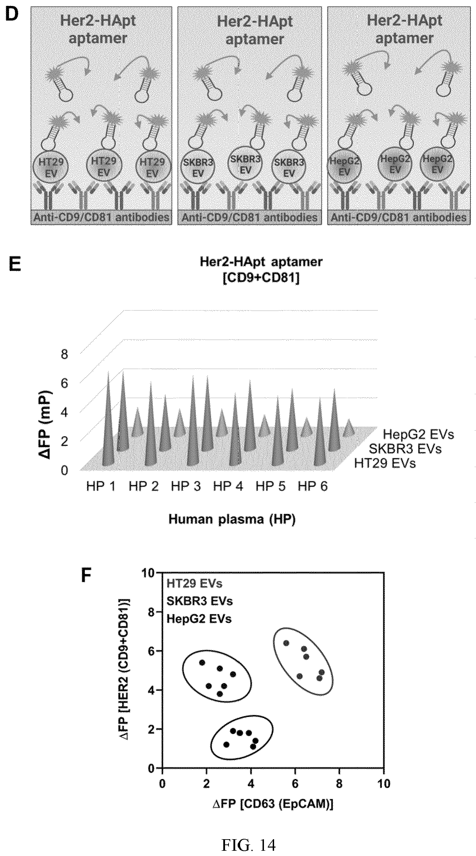

To ensure that the FluPADE results obtained using EVs prepared from cancer cell lines are not only relevant to the diagnostic settings of liquid biopsy but can also effectively overcome possible interferences from various components in individual patient plasma, the present applicant carefully prepared analytical samples by injecting the same number of EVs prepared from cancer cell lines into the plasma of six different blood donors. Accordingly, EVs prepared from human colorectal cancer cells (HT29), breast cancer cells (SKRB3), and liver cancer cells (HepG2) (1.0×10⁹ EV/mL) were added to the plasma of six donors, with the EV concentration in the plasma being 9.0×10⁹ EV/mL. The ratio of cancer cell line-secreted EVs to plasma EVs is 1:10 (v/v), with the total EV concentration in all 18 samples being the same, at 1.0×10¹⁰ EVs/mL. Using fluorescently labeled EV markers (CD63) or fluorescent cancer cell markers (HER2), EVs from three different types of solid cancers were detected, each added to the plasma from six blood donors, as shown in Figs. 14B and 14D. Interestingly, even though there are inter-sample differences among EVs from the same cell source, changes in fluorescence polarization based on CD63 aptamer detection can differentiate EVs affixed with the anti-EpCAM antibody from three different cancer cells (FIG. 14C). To test the reliability and stability of this assay method, the present invention designed a set of cross-tests, where antibodies corresponding to two EV markers (CD9 and CD81) were used to affix EVs from three different cancer sources, and the corresponding aptamer was used for the cancer biomarker HER2. As shown in FIG. 14E, in human plasma, the ΔFP for the HER2-HApt aptamer detecting HT29 sourced EVs is higher than the ΔFP for detecting SKBR3 sourced EVs. Most importantly, when the ΔFP for single analytes from these two groups of cross-tests is plotted in one graph (FIG. 14F), the variations in three cancer sourced EVs from three different cell sources are very distinct.

These results further illustrate the vast potential of FluPADE in the medical diagnostic field, as it can not only detect cancer-sourced EVs with high sensitivity but can also differentiate different EV populations that have the same cancer biomarker but vary in the biomarker abundance of EV surface .

A second aspect of the present invention provides an application of the siad aptamer-based fluorescence polarization detection method for extracellular vesicles, which is used for the qualitative and quantitative analysis of extracellular vesicles secreted by cancer cells.

In some preferred embodiments, the said cancer cells originate from any of the following: a colorectal cancer, a breast cancer, a hepatocellular carcinoma, a gastric cancer, a pancreatic cancer, an esophageal cancer, a nasopharyngeal carcinoma, a laryngeal cancer, an endometrial cancer, a lung cancer, a head and neck cancer, a kidney cancer, a bladder cancer, a thyroid cancer, a skin cancer, an ovarian cancer, a cervical cancer, a prostate cancer, or a penile cancer.

In some preferred embodiments, the detection method can differentiate EVs secreted by cancer cells from different primary sites, which include the intestine, breast, liver, stomach, pancreas, esophagus, epidermis, soft tissues of the skin, ovaries, cervix, prostate, or penis.

In some preferred embodiments, the detection method can differentiate the EVs secreted by cancer cells at different growth stages. These growth stages include the in-situ cancer stage, regional lymph node metastasis stage, and distant metastasis stage. Preferably, the detection method can distinguish the extracellular vesicles secreted by cancer cells at different growth stages during anti-cancer treatment.

In some preferred embodiments, the detection method can be directly performed on automated biochemical analyzers in clinical laboratories without the need for special equipment or customized instruments.

In some preferred embodiments, the detection method can be directly performed on automated immunoanalyzers in clinical laboratories without the need for special equipment or customized instruments.

### Beneficial Effects:

the present application uses aptamers as the probing substance and employs fluorescence polarization to detect extracellular vesicles secreted by cancer cells. It can effectively differentiate extracellular vesicles that contain the same biomarker protein but originate from different cell populations, making it suitable for qualitative and quantitative analysis in cancer in vito liquid biopsies. Moreover, the present invention can specifically detect extracellular vesicles secreted by cancer cells in the blood. The detection process is not affected by interference from free tumor marker proteins in the blood, tumor cell membrane fragments, or fragments of the extracellular vesicle membranes from tumor cells, ensuring accurate and effective results.

### BRIEF DESCRIPTION OF THE DRAWINGS

FIG. 1 is a schematic diagram of the fluorescence polarization detection steps for EVs derived from cancer cells;
FIG. 2 is a schematic diagram of the working principle of the nucleic acid aptamer-based fluorescence polarization detection method for extracellular vesicles;
FIG. 3 shows characteristics of EVs from HT29, SKBR3, HepG2, HEK293, and MDA-MB-231 cells with the HER2 gene knocked out; specifically, figure A thereof shows the EV particle size distribution measured by nanoparticle tracking analysis; figure B thereof shows the morphology of EVs observed under a scanning electron microscope; figure C shows the test results of the immunoblot tests performed using Alix, CD63, calnexin, EpCAM, and HER2; figure D shows the expression of surface biomarker proteins or cancer biomarkers (including CD9, CD63, CD81, EpCAM, and HER2) of EVs analyzed by flow cytometry; figure E is a histogram of the expression of surface marker proteins on EVs as detected and analyzed by the flow cytometry, where red color represents the background control (magnetic beads coated with IgG isotype control for immobilizing EVs), and blue color represents magnetic beads coated with the specific antibodies for immobilizing EVs.
In FIG. 4, figure A is a schematic diagram of EVs detected and immobilized using fluorescently-labeled anti-CD63 antibody (BioLegend, Cat No.: 353006); figure B shows EVs sourced from HT29 immobilized with biotinylated anti-EpCAM antibody under conditions of 16 hours in a cold chamber (BioLegend, Cat No.: 324216) and EVs sourced from SKRB3 immobilized using a 1:1 ratio of anti-CD9/CD81 antibodies (BioLegend, anti-CD9 antibody: Cat No.: 312112; anti-CD81 antibody: Cat No.: 349514). Herein, the biotin-labeled antibodies are coated in microwells coated with streptavidin (Thermo Fisher Scientific, Cat No.: 15503). Immobilized EVs undergo treatment with either 1% Triton X-100 or saline solution (p<0.0001); figure C shows the optimization of concentration for biotinylated anti-EpCAM antibody for HT29-sourced EVs and biotinylated anti-CD9/CD81 antibody for SKBR3-sourced EVs in comparison to the fluorescence intensity of EVs immobilized with 5µg/mL antibody, p<0.05; figure D shows the optimization of incubation time for biotinylated anti-EpCAM antibody for immobilizing HT29-sourced EVs and biotinylated anti-CD9/CD81 antibody for immobilizing SKBR3-sourced EVs in streptavidin-coated microwells; figure E shows optimization of immobilizing time for HT29-sourced EV/anti-EpCAM antibody or SKBR3-sourced EV/anti-CD9/CD81 in comparison to the fluorescence intensity of EVs incubated for 4 hours, p<0.05; the data is represented as the mean ± standard deviation, n=3.
FIG. 5 shows the binding affinity of antibodies and nucleic acid aptamers to immobilized EVs and the fluorescence polarization properties when using antibodies or aptamers on EVs; figure A shows the binding curve and apparent dissociation constant (K_{D}) of immobilizing the CD63 antibody to HT29-sourced EVs; figure B shows the binding curve and apparent dissociation constant (K_{D}) for immobilizing the CD63-BP aptamer to HT29-sourced EVs; figure C shows the difference in fluorescence polarization when immobilizing HT29-sourced EVs with anti-CD63 antibody or CD63-BP aptamer at concentrations of 5nM, K_{D}, and 2K_{D}; figure D shows the binding curve and K_{D} of immobilizing the anti-HER2 antibody to SKBR3-sourced EVs; figure E shows the binding curve and K_{D} for the HER2-HApt aptamer to SKBR3-sourced EVs; figure F presents the difference in fluorescence polarization when immobilizing SKBR3-sourced EVs with anti-HER2 antibody and HER2-HApt aptamer at concentrations of 5nM, K_{D}, and 2K_{D}; the data is represented as the mean ± standard deviation, n=3.
FIG. 6 shows Determination of the optimal concentration of aptamers in fluorescence polarization assays; figure A shows the signal-to-noise ratio of parallel and vertical fluorescence intensities for CD63-BP aptamer, HER2-HApt aptamer, and HER2-2A aptamer at different concentrations; figure B shows the relationship between fluorescence polarization and concentration for the aptamers at different concentrations; the data is represented as the mean ± standard deviation, n=3.
FIG. 7 shows determination of the optimal incubation time between the aptamer and immobilized EVs to achieve the best fluorescence polarization signal; figure A shows the action of the CD63-BP aptamer on HT29-sourced EVs; figure B shows the action of the HER2-HApt aptamer on SKRB3-sourced EVs; figure C shows the action of the HER2-2A aptamer on SKRB3-sourced Evs; the data is represented as the mean ± standard deviation, n=3.
FIG. 8 shows fluorescence polarization differences of CD63 and HEER2 aptamers in different samples; figure A shows the fluorescence polarization differences when a 5nM fluorescently labeled CD63-BP aptamer interacts with HT29-sourced EVs immobilized by the anti-EpCAM antibody among 6 samples; the fluorescently labeled aptamer does not bind to HT29-sourced EVs immobilized by CD63 and anti-EpCAM; the HT29-sourced EVs immobilized by the said anti-EpCAM are sourced from Triton X-100 lysis (negative control matched with anti-EpCAM antibody type), EVs sourced from HEK293 with non expression of EpCAM, or free EpCAM protein (50nM); figure B shows the fluorescence polarization differences of the 5nM fluorescently labeled HER2-HApt-BP aptamers among 5 different samples: anti-CD9/CD81 antibodies immobilize SKRB3-sourced Evs; the fluorescently labeled aptamer does not bind to SKRB3-sourced EVs immobilized with HER2 and anti-CD9/CD81 antibodies, SKRB3-sourced EVs immobilized with anti-CD9/CD81 antibodies and lysed with Triton X-100 , SKRB3 source cells cultured in wells coated with anti-IgG antibody, and HER2 gene-knockout MDA-MB-231-sourced EVs (HER2-negative EVs). figure C shows the fluorescence polarization differences of the 5nM fluorescently labeled HER2-2A aptamers among the aforementioned 5 different samples, the data is represented as the mean ± standard deviation, n=3.
FIG. 9 shows changes of fluorescence polarization signal with EV concentration. The LOD and LDR of the FluPADE detection method are determined based on a linear curve of the internal fluorescence signal changing with log10(lg) EV concentration. The EV concentrations are determined in PBS (Figures A, C, E) or human plasma (1:10, v/v; Figures B, D, F) based on biopsy of the CD63-BP aptamer (figure A-B), HER2-HApt aptamer (figure C-D), or HER2-2A aptamer (figure E-F). All FP and ΔFP values are presented in millipolarization (mP) units; all FP values are presented in millipolarization (mP) units, with each sample's FP value being calculated from the average of three different wells, and each well's FP value being the average of three measurements for that well.
FIG. 10 shows background signal detection of EV marker proteins and cancer biomarkers in human plasma EV measurement; in figure A the red spectrum represents fluorescence intensities for the fluorescently labeled anti-EpCAM antibody, anti-CD63 antibody, anti-CD81 antibody, and anti-HER2 antibody during separating EVs from human plasm at excitation wavelengths of 520~660nm (emission at 535nm) when using the anti-EpCAM antibody to detect EpCAM and CD63, and using the anti-CD9/CD81 antibody to detect CD81 and HER2 ; the blue spectrum shows the fluorescence intensity when using the IgG isotype control antibody to separate EVs from human plasma. During separation of EVs from human plasma using the anti-CD9/CD81 antibody, the fluorescence spectrum (red) of the PE-anti-CD9 antibody at excitation wavelengths 570nm~690nm (emission wavelength at 575nm) and the fluorescence intensity spectrum (blue) using the IgG isotype control antibody for separation of EVs from human plasma are also recorded. figure B shows respectively measured fluorescence intensities of EVs separated from human plasma using the anti-EpCAM antibody (ex: 485nm, em: 535nm), anti-CD63 antibody (ex: 485nm, em: 535nm), anti-CD9 antibody (ex: 560 nm, em: 575nm), anti-CD81 antibody (ex: 485nm, em: 535nm), and anti-HER2 antibody (ex: 485nm, em: 535nm). The data is represented as the mean ± standard deviation, n=3.
FIG. 11 shows LOD and LDR based on fluorescence intensity detection using aptamers; specifically, it shows the relationship diagram between EV concentration and internal fluorescence intensity and the results of LOD and LDR when measuring HT29-sourced EVs using the CD63-BP aptamer (figures A-B), and measuring SKRB3-sourced EVs using the HER2-HApt aptamer (figures C-D) or HER2-2A aptamer (figures E-F); the EVs sourced from the cell lines are diluted with PBS (Figures A, C, E) and the human plasma (Figures B, D, F).
FIG. 12 shows the LOD and LDR for fluorescence intensity detection based on antibodies; specifically, using the anti-CD63 antibody (figures A-B) to detect HT29-sourced EVs and using the anti-HER2 antibody (figures C-D) to detect SKRB3-sourced EVs, showing the relationship between EV concentration and internal fluorescence intensity; the cell-line-sourced EVs were diluted with PBS (Figures A, C) and human plasma (Figures B, D).
FIG. 13 shows sensitivity of the fluorescence polarization method based on aptamers for detecting cancer-sourced Evs; specifically, the change in aptamer fluorescence polarization signal under the effect of linear concentration of cancer-sourced EVs. Figure A shows the ratio of EpCAM-positive HT29-sourced EVs to EpCAM-negative HEK293-sourced EVs detected by 5.0 nM CD63-BP aptamer. Figures B-C show the ratio of HER2-positive SKBR3-sourced EVs to HER2-negative MDA-MB-231-sourced EVs detected by 5.0 nM HER2-HApt aptamer or HER2-2A aptamer, respectively. The data is presented as mean ± standard deviation, n=3.
FIG. 14 shows that FluPADE can distinguish EVs from different sources based on dual fluorescence polarization analysis of biomarkers. Figure A shows the first row of figures shows the expression level of CD63 in HT29-sourced EVs, SKBR3-sourced EVs, and HepG2-sourced EVs separated by anti-EpCAM antibody-coated magnetic beads; the second row of figures shows the expression level of HER2 in the HT29-sourced EVs, SKBR3-sourced EVs, and HepG2-sourced EVs separated by anti-CD9/CD81 antibody-coated magnetic beads. Red color indicates the background fluorescence of EVs cultured with IgG isotype control antibody-coated magnetic beads, while blue color indicates the fluorescence intensity of EVs separated by anti-EpCAM antibody-coated magnetic beads (first row) and the fluorescence intensity of EVs separated by anti-CD9/CD81 antibody-coated magnetic beads (second row). Figure B shows a schematic diagram of FluPADE detection using the CD63-BP aptamer to detect EVs from three different cancer cell lines separated from human plasma by the anti-EpCAM antibody, sourced from six blood donors. Figure C shows the fluorescence polarization signal differences of the CD63-BP aptamer acting on EVs from three different cancer cell lines separated by the anti-EpCAM antibody from the blood plasma which is sourced from six blood donors. Figure D shows a schematic diagram of FluPADE detection using the HER2-HApt aptamer to detect EVs from three different cancer cell lines separated from human plasma by the anti-CD9/CD81 antibody, the blood plasma being sourced from six blood donors. Figure E shows the fluorescence polarization signal differences of the HER2-HApt aptamer acting on EVs from the three different cancer cell lines separated by the anti-CD9/CD81 antibody from the blood plasma, which is sourced from six blood donors. Figure F shows a clustering diagram of ΔFP for HT29-sourced EVs, SKBR3-sourced EVs, and HepG2-sourced EVs detected by CD63 aptamer and HER2-HApt aptamer (CD63 aptamer: Figure 14C; HER2-HApt aptamer: Figure 14E); the data is presented as mean ± standard deviation, n=3.

### DETAILED DESCRIPTION OF EXEMPLARY EMBODIMENTS

### Example 1.

A CD63-BP aptamer based fluorescence polarization detection method for extracellular vesicles in PBS includes:
S1: immobilizing HT29-sourced EVs and SKBR3-sourced EVs in microwells by using 8.0 µg/mL of anti-EpCAM antibody and anti-CD9/CD81 antibody (in a 1:1 mass ratio). Specifically, biotin-labeled antibodies are placed in the streptavidin-coated microwells, incubated at a room temperature for 30 minutes and washed. The EVs are then incubated at 4□ for over 16 hours.
S2: adding 100µL of 5.0nM fluorescently labeled CD63-BP aptamer in PBS (a phosphate buffer solution containing 1.5 mM MgCl₂ after being filtered with a filter membrane of 0.2 µm) to each microwell, incubating them on a shaker (Thermoline Scientific, model: TL400) under a light-protected condition at a room temperature for 1 hour.
S3: reading FP signals using a multi functionalplate reader CLARIOstar Plus (BMG Labtech) equipped with an excitation filter at 485nm and an emission filter at 535nm.

FP control samples with free ligands are prepared using the same method mentioned above, specifically, an equal volume of PBS without EVs is added to the microwells.

### Example 2:

A HER2-HApt aptamer based fluorescence polarization detection method forextracellular vesicles in PBS includes:
S1: immobilizing HT29-sourced EVs and SKBR3-sourced EVs in microwells by using 8µg/mL of anti-EpCAM antibody and anti-CD9/CD81 antibody (in a 1:1 mass ratio) respectively. Specifically, biotin-labeled antibodies are placed in the streptavidin-coated microwells, incubated at a room temperature for 30 minutes and washed. The EVs are then incubated at 4□ for over 16 hours.
S2: adding 100µL of 5nM fluorescently labeled HER2-HApt aptamer in PBS (a phosphate buffer solution filtered through a filter membrane of 0.2 µm) to each microwell, and incubating them on a shaker (Thermoline Scientific, model: TL400) under a light-protected condition at a room temperature for 1 hour.
S3: reading FP signals byusing a multi functional plate reader CLARIOstar Plus (BMG Labtech) equipped with an excitation filter at 485nm and an emission filter at 535nm.
FP control samples with free ligands are prepared using the same method mentioned above, specifically, an equal volume of PBS without EVs is added to the microwells.

### Example 3.

A HER2A aptamer based fluorescence polarization detection method for extracellular vesicles in PBS includes:
S1: immobilizing HT29-sourced EVs and SKBR3-sourced EVs in microwells by using 8µg/mL of anti-EpCAM antibody and anti-CD9/CD81 antibody (in a 1:1 mass ratio). Specifically, biotin-labeled antibodies are placed in the streptavidin-coated microwells, incubated at a room temperature for 30 minutes and washed. The EVs are then incubated at 4□ for over 16 hours.
S2: adding 100µL of 5nM fluorescently labeled HER2A aptamer in PBS (a phosphate buffer solution filtered through a filter membrane of 0.2 µm ) to each microwell, and then incubating on a shaker (Thermoline Scientific, model: TL400) under a light-protected condition at a room temperature for 1.5 hours.
S3: reading FP signals by using a multi functional plate reader CLARIOstar Plus (BMG Labtech) equipped with an excitation filter at 485nm and an emission filter at 535nm.

FP control samples with free ligands are prepared using the same method mentioned above. Specifically, an equal volume of PBS without EVs is added to the microwells.

### Example 4.

A CD63-BP aptamer based fluorescence polarization detection method for extracellular vesicles in human plasma, has the same specific implementation as those in Example 1; the difference is in step S1, before immobilizing the EVs, a target amount of EVs is added to human plasma (in a 1:10 ratio, v/v). Subsequently, 8.0µg/mL of anti-EpCAM antibody and anti-CD9/CD81 antibody (in a 1:1 mass ratio) are respectively used to fix or immobilize the cell line-sourced EVs in human plasma in microwells.

FP control samples with free ligands are prepared in the same way as described above. Specifically, 100µL of F-PBS is added to 900µL of human plasma to serve as the FP control sample for human plasma measurement.

### Example 5.

A HER2-HApt aptamer based fluorescence polarization detection method for extracellular vesicles in human plasma, has the same specific implementation as those in Example 4; the difference is that in step S2, the CD63-BP aptamer is replaced with the HER2-HApt aptamer.

### Example 6.

A HER2A aptamer based fluorescence polarization detection method for extracellular vesicles in human plasma, has the same specific implementation as those in Example 4; the difference is in step S2, the CD63-BP aptamer is replaced with the HER2A aptamer, and the incubation time in S2 is 1.5 hours.

### Example 7.

A CD63-BP aptamer based fluorescence intensity detection method for extracellular vesicles in PBS, has the same specific implementation as those in Example 1; the difference is:
In said step S2, 100µL of PBS buffer solution containing 800nM fluorescently labeled CD63-BP aptamer is added, followed by the addition of 100µL of PBS with either 50nM of fluorescently labeled CD63 antibody or 50nM fluorescently labeled HER2 antibody, they are then incubated in the dark room or light-shielded room at a room temperature on a shaker (Thermoline Scientific, Model: TL400) for 1 hour.

In said step S3, after washing three times with 200µL wash buffer solution, fluorescence intensity is measured using the multi functional plate reader CLARIOstar Plus (BMG Labtech) under an excitation filter at 485nm and an emission filter at 535nm.

### Example 8.

A HER2-HApt aptamer based fluorescence intensity detection method for extracellular vesicles in PBS, has the same specific implementation as those in Example 7, the difference is that the CD63-BP aptamer in step S2 is replaced with the HER2-HApt aptamer.

### Example 9.

A HER2-HApt aptamer based fluorescence intensity detection method for extracellular vesicles in PBS, has the same specific implementation as those in Example 7, the difference is that the CD63-BP aptamer in step S2 is replaced with the HER2-2A aptamer.

### Example 10.

An anti-CD63 antibody based fluorescence intensity detection method for extracellular vesicles in PBS, has the same specific implementation as those in Example 7, the difference is that the CD63-BP aptamer in step S2 is replaced with the anti-CD63 antibody.

### Example 11.

An anti-HER2 antibody based fluorescence intensity detection method for extracellular vesicles in PBS, has the specific implementation as those in Example 7, the difference is that the CD63-BP aptamer in step S2 is replaced with the anti-HER2 antibody.

### Example 12.

A CD63-BP aptamer based fluorescence intensity detection method for extracellular vesicles in human plasma, has the same specific implementation as those in Example 7; the difference is in step S1, before immobilizing or immobilizing the EVs, a target amount of EVs is added to human plasma (at a 1:10 ratio, v/v). Then, 8µg/mL of anti-EpCAM antibody and anti-CD9/CD81 antibody (in a 1:1 mass ratio) are used to immobilize the cell line-sourced EVs of the human plasma in the microwells.

FP control samples with free ligands are prepared using the same method described above, specifically adding 100µL of F-PBS to 900µL of human plasma as a control sample for human plasma measurement.

### Example 13.

A HER2-HApt aptamer based fluorescence intensity detection method for extracellular vesicles in human plasma, has the same specific implementation as those in Example 12, the difference is that the CD63-BP aptamer in step S2 is replaced with the HER2-HApt aptamer.

### Example 14.

A HER2-2A aptamer based fluorescence intensity detection method for extracellular vesicles in human plasma, has the same specific implementation as those in Example 12, the difference is that the CD63-BP aptamer in step S2 is replaced with the HER2-2A aptamer.

### Example 15.

An anti-CD63 antibody based fluorescence intensity detection method for extracellular vesicles in human plasma, has the same specific implementation as those in Example 12, the difference is that the CD63-BP aptamer in step S2 is replaced with the anti-CD63 antibody.

### Example 16.

An anti-CD63 antibody based fluorescence intensity detection method for extracellular vesicles in human plasma, has the specific implementation as those in Example 12, the difference is that the CD63-BP aptamer in step S2 is replaced with the anti-HER2 antibody.

The LOD for the above examples is determined by the EV concentration obtained by the test signal, where the EV concentration equals the signal of the control sample plus three times the standard deviation of the control sample results. The linear dynamic range is defined by the linear regression of the EV concentration signal. Measurement results are presented in Table 1.

**Table 1. Summary of LOD and LDR results for methods in Examples 1~16.**

| Example | Method | Detection probes | LOD in PBS EVs/mL | LDR in PBS Evs/mL |
|---|---|---|---|---|
| 1 | FluPADE | CD63-BP aptamer | 5.0×10⁶ | 5.0×10⁸-2.0×10¹⁰ |
| 2 | FluPADE | HER2-HApt aptamer | 3.0×10⁷ | 5.0×10⁸-2.0×10¹⁰ |
| 3 | FluPADE | HER2-2A aptamer | 1.0×10⁷ | 2.0×10⁹-2.0×10¹⁰ |
| 7 | Fl | CD63-BP aptamer | 2.0×10⁸ | 3.0×10⁸-2.0×10⁹ |
| 8 | Fl | HER2-HApt aptamer | 5.0×10⁸ | 1.10×10⁹-2.0×10¹⁰ |
| 9 | Fl | HER2-2A aptamer | 2.0×10⁸ | 3.0×10⁸-2.0×10⁹ |
| 10 | Fl | anti-CD63 antibody | 1.0×10⁷ | 5.0×10⁷-1.0×10⁹ |
| 11 | Fl | anti-HER2 antibody | 1.0×10⁹ | 2.0×10⁹-2.0×10¹⁰ |

| Example | Method | Detection probes | LOD in human plasma EVs/mL | LDR in human plasma Evs/mL |
|---|---|---|---|---|
| 4 | FluPADE | CD63-BP aptamer | 5.0×10⁷ | 5.0×10⁸-1.0×10¹⁰ |
| 5 | FluPADE | HER2-HApt aptamer | 5.0×10⁷ | 8.0×10⁷-1.0×10¹⁰ |
| 6 | FluPADE | HER2-2A aptamer | 3.0×10⁷ | 2.0×10⁸-1.0×10¹⁰ |
| 12 | Fl | CD63-BP aptamer | 5.0×10⁸ | 1.0×10⁹-1.0×10¹⁰ |
| 13 | Fl | HER2-HApt aptamer | 1.0×10⁹ | 3.0×10⁹-1.0×10¹⁰ |
| 14 | Fl | HER2-2A aptamer | 8.0×10⁸ | 1.0×10⁹-1.0×10¹⁰ |
| 15 | Fl | anti-CD63 antibody | 5.0×10⁷ | 1,0×10⁸-2.0×10⁹ |
| 16 | Fl | anti-HER2 antibody | 3.0×10⁹ | 5.0×10⁹-1.0×10¹⁰ |

## Claims

1. A aptamer-based fluorescence polarization detection method for extracellular vesicles, **characterized in that**, it comprises following steps:
S1. immobilizing EVs by interacting with antibodies against surface-biomaker proteins of EVs or surface cancer markers of EVs; rapidly washing them to remove free EVs, proteins, membrane fragments, and lipids;
S2. respectively adding aptamers that are matched with EV markers or cancer cell markers therein and cultivating them, and the aptamers being fluorescently labeled; and
S3. performing fluorescence polarization detection on products from Step S2 to achieve qualitative and quantitative analysis of EVs secreted by cancer cells, without requiring washing during operation;
the EV markers comprise at least one of CD9, CD63, and CD81;
the cancer cell markers comprise EpCAM and/or HER2;
the aptamers comprise at least one of CD63-BP, HER2-HApt, and HER2-2A.

2. The aptamer-based fluorescence polarization detection method for extracellular vesicles according to claim 1, **characterized in that**, in the step S1, when the EVs are sourced from HT29, the antibody is biotinylated anti-human EpCAM antibody, and the concentration of the antibody is 2.0 to 15.0µg/mL.

3. The aptamer-based fluorescence polarization detection method for extracellular vesicles according to claim 1, **characterized in that**, in the step S1 , when the EVs are sourced from SKBR3, the antibody is biotinylated anti-human CD9/CD81 antibody, and the concentration of the antibody is 2.0 to 15.0µg/mL.

4. The aptamer-based fluorescence polarization detection method for extracellular vesicles according to claim 2 or 3, **characterized in that**, the step S1 is performed in a microwell plate, using microwells coated with streptavidin for antibody capture, and being followed by EV immobilizing;
a time for capture antibody is 0.1 to 1.5 hours; and an immobilizing time is 4 to 20 hours, and an immobilizing temperature is 4°C.

5. The aptamer-based fluorescence polarization detection method for extracellular vesicles according to claim 1, **characterized in that**, the aptamers undergo a folding process before use, wherein the specific steps comprise: diluting the aptamers to a target concentration using a phosphate buffer solution added with 0.5 to 2.0 mM MgCl₂, denaturing them at 90 to 98°C for 2 to 10 minutes, incubating them on ice or at room temperature for 5 to 20 minutes, and then refolding them at 35 to 38°C for 10 to 30 minutes.

6. The aptamer-based fluorescence polarization detection method for extracellular vesicles according to claim 1 or 5, **characterized in that**, the target concentration of the aptamers is 1 to 8nM.

7. The aptamer-based fluorescence polarization detection method for extracellular vesicles according to claim 6, **characterized in that**, the step S2 specifically comprises adding 60 to 140 µL of a buffer solution containing fluorescently labeled aptamers to product obtained from the step S1, and incubating the microwell plate on a shaker at a room temperature in the dark for 0.5 to 2 hours.

8. The aptamer-based fluorescence polarization detection method for extracellular vesicles according to claim 7, **characterized in that**, the buffer solution comprises a synthetic buffer solution or human plasma;
preferably, the human plasma is from donors having any one blood type of A, B, AB, O, Rh+, or Rh- ;
preferably, the human plasma is from donors aged from 0 to 120 years;
preferably, the human plasma is from healthy or non-healthy individuals;
further preferably, the human plasma is from non-healthy individuals, and non-healthy individuals are preferably tumor patients.

9. The aptamer-based fluorescence polarization detection method for extracellular vesicles according to claim 1, **characterized in that**, the fluorescence polarization signal in the step S3 is read using a multifunctional plate analyzer; and the multifunctional plate analyzer is equipped with an excitation filter at 475 to 490nm and an emission filter at 520 to 565nm.

10. The aptamer-based fluorescence polarization detection method for extracellular vesicles according to claim 9, **characterized in that**, when the aptamer is CD63-BP, LOD of the detection method is LOD ≤5 × 10⁷EVs/mL, and LDR) is 5 × 10⁸ to 2 × 10¹⁰ EVs/mL; when the aptamer is HER2-HApt, the LOD of the detection method is LOD ≤5 × 10⁷EVs/mL, and the LDR is 8 × 10⁷ to 2 × 10¹⁰EVs/mL; and when the nucleic acid aptamer is HER2-2A, the LOD of the detection method is LOD ≤3 × 10⁷EVs/mL, and the LDR is 2 × 10⁸ to 2 × 10¹⁰EVs/mL.

11. An application of the aptamer-based fluorescence polarization detection method for extracellular vesicles according to claim 1, **characterized in that**, the detection method is applied for qualitative and quantitative analysis of extracellular vesicles secreted by cancer cells.

12. The application of the aptamer-based fluorescence polarization detection method for extracellular vesicles according to claim 1, **characterized in that**, the cancer cells originate from any one of a colorectal cancer, a breast cancer, a hepatocellular cancer, a gastric cancer, a pancreatic cancer, an esophageal cancer, a nasopharyngeal cancer, a laryngeal cancer, an endometrial cancer, a lung cancer, a head and neck cancer, a kidney cancer, a bladder cancer, a thyroid cancer, a skin cancer, an ovarian cancer, a cervical cancer, a prostate cancer, and a penile cancer.

13. The application of the aptamer-based fluorescence polarization detection method for extracellular vesicles according to claim 11 or 12, **characterized in that**, the detection method can distinguish extracellular vesicles secreted by cancer cells from different primary sites; and the primary sites comprise any one of intestine, breast, liver, stomach, pancreas, esophagus, lung, gallbladder, bladder, thyroid, ovary, cervix, prostate, and penis.

14. The application of the aptamer-based fluorescence polarization detection method for extracellular vesicles according to claim 11, **characterized in that**, the detection method can distinguish extracellular vesicles secreted by cancer cells at different stages of growth; and the growth stages comprise any one of in situ cancer stage, regional lymph node metastasis stage, and distant metastasis stage; preferably, the detection method can distinguish extracellular vesicles secreted by cancer cells during different stages of anticancer treatment or extracellular vesicles produced by cancer cells having drug-resistant properties following anticancer treatment.

15. The application of the aptamer-based fluorescence polarization detection method for extracellular vesicles according to claim 11, **characterized in that**, the detection method can be directly conducted on an automated biochemical analyzer in a clinical laboratory without requiring special equipment or customized instruments.

16. The application of the aptamer-based fluorescence polarization detection method for extracellular vesicles according to claim 11, **characterized in that**, the detection method can be directly conducted on an automated immunoassay analyzer in a clinical laboratory without requiring special equipment or customized instruments.
